# EUROPÄISCHE PATENTSCHRIFT

(11) **EP 3 033 134 B1**
(45) Veröffentlichungstag und Bekanntmachung des Hinweises auf die Patenterteilung: **08.05.2019**
(21) Anmeldenummer: 14750201.7
(22) Anmeldetag: 07.08.2014
(51) Int. Cl.: A61M 25/06

(54) **SICHERHEITSCLIP FÜR EINE SELDINGERKANÜLE**
SAFETY CLIP FOR A SELDINGER CANNULA
CLIP DE SÉCURITÉ POUR CANULE DE SELDINGER

(30) Priorität: 15.08.2013 DE 102013216228
(43) Veröffentlichungstag der Anmeldung: 22.06.2016
(73) Patentinhaber: B. Braun Melsungen AG, 34212 Melsungen (DE)
(72) Erfinder: WEISS, André, 34302 Guxhagen (DE)
(74) Vertreter: Patentanwälte Ruff, Wilhelm, Beier, Dauster & Partner mbB
(86) Internationale Anmeldenummer: PCT/EP2014/067008
(87) Internationale Veröffentlichungsnummer: WO 2015/022260

(56) Entgegenhaltungen:
- DE-A1-102008 045 692
- US-A1- 2004 133 167

## Beschreibung

Die Erfindung betrifft eine Kanüle mit einem langgestreckten, an beiden Enden offenen Rohr zur Aufnahme eines langgestreckten Elements in Form eines Drahtes, eines Fadens oder eines Stabes. Bei dem langgestreckten Element kann es sich insbesondere um einen Führungsdraht und bei der Kanüle um eine Seldingerkanüle zur Positionierung des Führungsdrahtes handeln.

Ein Führungsdraht wird zur Einführung und Positionierung eines Patienten-Katheters verwendet. Ein oft verwendeter Führungsdraht ist ein Seldingerdraht, der mit Hilfe einer Seldingerkanüle in den Patienten eingeführt wird. Die Seldingerkanüle weist hierzu ein langgestrecktes Rohr auf, dessen einander gegenüberliegende Enden offen sind, um den Führungsdraht durch das Rohr hindurchschieben zu können. Das eine Ende der Seldingerkanüle ist zum Einführen in den Patienten mit einer Spitze versehen. Das andere, rückwärtige Ende der Kanüle ist nicht dazu vorgesehen, in den Patienten eingeführt zu werden.

Zum Platzieren des Führungsdrahtes in dem Patienten wird zunächst die Seldingerkanüle eingeführt, in dem der Patient mit der distalen Spitze der Seldingerkanüle punktiert wird. Sobald das in den Patienten eingeführte Ende der Seldingerkanüle korrekt in dem Gefäß platziert wird, in das der Führungsdraht eingeführt werden soll, wird der Führungsdraht durch das rückwärtige, nicht in den Patienten eingeführte Ende der Seldingerkanüle eingeschoben. Der Führungsdraht wird vollständig durch die Seldingerkanüle hindurch in das Gefäß eingeschoben und an den gewünschten Ort vorgeschoben. Sobald der Führungsdraht an der gewünschten Stelle platziert ist, wird die Seldingerkanüle aus dem Patienten herausgezogen, wobei der Fürhungsdraht in dem Patienten verbleibt.

Beim Herausziehen der Seldingerkanüle aus dem Patienten soll die distale Spitze verdeckt werden, um versehentliche Stichverletzungen zu vermeiden. Hierzu ist es bekannt, einen die Seldingerkanüle außen umschließenden Sicherheitsclip vorzusehen, der entlang der Seldingerkanüle verschiebbar ausgebildet ist. Nach dem Herausziehen der Seldingerkanüle aus dem Patienten wird der Sicherheitsclip manuell von dem Bediener über das distale Ende der Seldingerkanüle hinaus vorgeschoben, um das distale Ende zu verdecken und zu schützen.

Das Beispiel der Seldingerkanüle ist eines von vielen möglichen Beispielen der Verwendung eines langgestreckten Elements, das durch eine Kanüle vorgeschoben wird. Bei der Kanüle kann es sich um eine medizinische Kanüle oder um einen Katheter handeln. Das langgestreckte Element kann irgendein in einen Patienten einzuführendes Element in Schnur-, Stab- oder Draht-Form sein.

Siehe zum Beispiel DE 10 2008 045692 A1.

Der Erfindung liegt die Aufgabe zugrunde, die Bedienung des Sicherungsmechanismus einer Kanüle zu verbessern.

Die erfindungsgemäße Kanüle ist definiert durch die Merkmale von Patentanspruch 1. Der Sicherheitsclip ist dazu ausgebildet, mit dem langgestreckten Element in eine magnetische Wechselwirkung zu treten, die zu einer magnetischen Anziehung oder Abstoßung zwischen dem Sicherheitsclip und dem langgestreckten Element führt. Bei einer Variante weist der Sicherheitsclip ein magnetisch mit dem langgestreckten Element zusammenwirkendes Magnetelement oder magnetisches Material auf. Alternativ kann das langgestreckte Element ein magnetisch mit dem Sicherheitsclip zusammenwirkendes Magnetelement oder magnetisches Material aufweisen. Das Magnetelement kann zum Beispiel am Ende des langgestreckten Elements vorgesehen sein oder das langgestreckte Element kann vollständig aus einem magnetischen Material, zum Beispiel als Draht, bestehen. Dabei ist denkbar, dass sowohl der Sicherheitsclip als auch das langgestreckte Element ein Magnetelement aufweisen. Alternativ kann der Sicherheitsclip ein Magnetelement und das langgestreckte Element ein magnetisches Material oder umgekehrt aufweisen. Bei den Magnetelementen kann es sich um einen Dauermagneten aus Aluminium-Nickel-Kobalt, einem Ferrit, Bismanol oder Neodym-Eisen-Bor oder um einen Elektromagneten handeln.

Erfindungsgemäß wird ermöglicht, dass beim Verschieben der Kanüle gegenüber dem langgestreckten Element die magnetische Wechselwirkung zwischen dem Sicherheitsclip und dem langgestreckten Element eine Kraft auf den Sicherheitsclip ausübt. Der Sicherheitsclip wird so gegenüber der Kanüle verschoben. Beim Zurückziehen der Kanüle gegenüber dem langgestreckten Element verschiebt die Magnetkraft den Sicherheitsclip bis über die distale Spitze der Kanüle, wenn diese vollständig von dem langgestreckten Element abgezogen wird. Ein manuelles Verschieben des Sicherheitsclips ist nicht erforderlich. Die magnetische Wechselwirkung zwischen dem Sicherheitsclip und dem langgestreckten Element führt zu einem automatischen Verdecken der Kanülenspitze, wenn die Kanüle vollständig von dem langgestreckten Element abgezogen wird.

Vorzugsweise ist der Sicherheitsclip mit mindestens einem Federarm versehen, der in Richtung auf das Rohrinnere der Kanüle derart vorgespannt ist, dass beim Verschieben über das Rohrende hinaus der Federarm in seine ursprüngliche Lage zurückfedert und dabei das Rohrende verschließt oder abdeckt.

Die Kanüle kann im Bereich ihres distalen Endes mit einem Vorsprung oder einer Vertiefung versehen sein, die mit dem Sicherheitsclip zusammengreift, um dessen Vorschub entlang der Kanüle zu stoppen. Der Vorsprung oder die Vertiefung bilden somit einen Anschlag, der ein weiteres Vorschieben des Sicherheitsclips blockiert. Der Sicherheitsclip kann dadurch nur soweit vorgeschoben werden, bis das distale Ende der Kanüle verschlossen ist, ohne jedoch den Sicherheitsclip vollständig von der Kanüle abzuziehen.

Das rückwärtige, proximale Ende der Kanüle ist vorteilhafterweise mit einem aufgeweiteten Außendurchmesser versehen, um den Vorschub des Sicherheitsclips zu stoppen. Der aufgeweitete Außendurchmesser bildet einen rückwärtigen Anschlag für den Sicherheitsclip und kann beispielsweise als Trichter zum erleichterten Einführen des langgestreckten Elements in das rückwärtige Ende der Kanüle dienen. Der Sicherheitsclip kann dadurch nicht über das rückwärtige, proximale Ende der Kanüle hinaus abgezogen werden.

Das erfindungsgemäße langgestreckte Element zur Verwendung mit der erfindungsgemäßen Kanüle kann mit einem mit dem Magnetelement des Sicherheitsclips magnetisch zusammenwirkenden Element versehen sein. Bei dem Element kann es sich beispielsweise um einen Permanentmagneten handeln. Das magnetische Element des langgestreckten Elements ist dabei vorzugsweise an dessen rückwärtigen proximalen Ende angeordnet. Ein Abziehen der Kanüle von dem langgestreckten Element über dessen proximales Ende hinaus führt dann dazu, dass das magnetische Element des langgestreckten Elements eine Kraft auf den Sicherheitsclip ausübt und den Sicherheitsclip in Richtung der distalen Spitze der Kanüle verschiebt.

Im Folgenden wird anhand der Figuren ein Ausführungsbeispiel der Erfindung näher erläutert. Es zeigen:
- Fig. 1: eine schematische Darstellung des Ausführungsbeispiels beim Vorschieben des Führungsdrahtes,
- Fig. 2: die Ansicht nach Fig. 1 beim Zurückziehen der Kanüle und
- Fig. 3: die Ansicht nach Fig. 1 nach dem Zurückziehen der Kanüle.

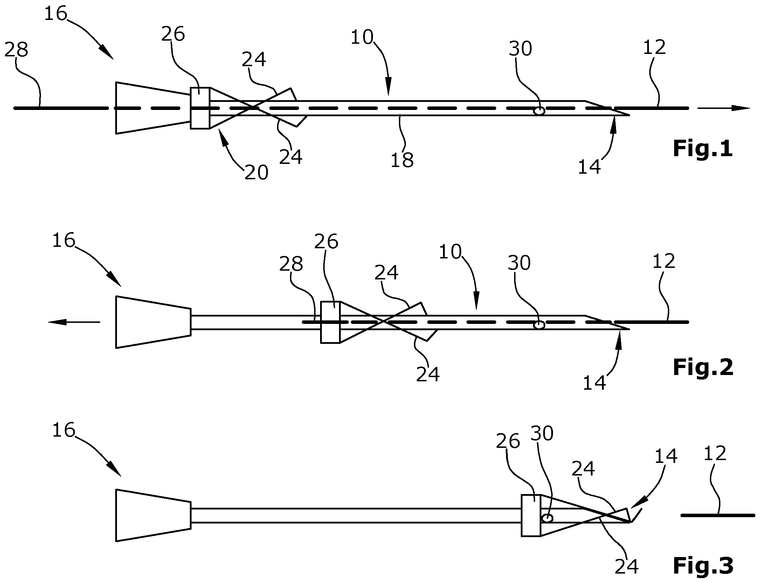

Die Kanüle 10 ist eine Seldingerkanüle mit einem zylindrischen Rohr 18, das ein vorderes, zum Einführen in den Patienten ausgebildetes distales Ende 14 und ein rückwärtiges, nicht in den Patienten einzuführendes proximales Ende 16 aufweist. Das rückwärtige Ende 16 ist trichterförmig aufgeweitet, um das Einführen des langgestreckten Elements 12 in die Seldingerkanüle 10 zu erleichtern. Das langgestreckte Element ist ein Führungsdraht in Form eines Seidingerdrahts. In Fig. 1 ist der Führungsdraht 12 vollständig durch die Seldingerkanüle hindurchgeführt, das heißt er ragt aus den beiden Enden 14, 16 hinaus.

Auf die Seldingerkanüle 10 ist ein Sicherheitsclip 20 aufgeschoben. Der Sicherheitsclip 20 ist außen auf das Rohr 18 aufgeschoben und entlang des Rohres 18 gleitend verschiebbar. Der Sicherheitsclip 20 weist eine magnetische Basis 26 aus einem permanent magnetischen Material auf. An der Basis sind zwei Federarme 24 angeordnet, die nach dem Prinzip einer Schere oder Zange gegeneinander und in Richtung auf das Rohrinnere elastisch vorgespannt sind, um das distale Ende 14 der Seldingerkanüle 10 zu verschließen, wenn die Federarme 24 über das distale Ende 14 hinausgeschoben werden, wie in Fig. 3 dargestellt.

Im Bereich des distalen Endes 14 ist das Rohr 18 mit einer Vertiefung 30 in Form einer Sicke versehen, die einen Anschlag für die Basis 26 des Sicherheitsclips 20 bildet. Die Basis 26 kann in distaler Richtung nicht über die Vertiefung 30 hinausgeschoben werden. Der Abstand der Vertiefung 30 bis zum distalen Ende 14 des Rohres ist geringer als die Länge jedes Federarms 24. Dadurch verschließen die Federarme 24 das distale Ende 14, wenn die Basis 26 an der Vertiefung 30 anliegt.

Nach dem Einführen des Führungsdrahtes 12 durch die Seldingerkanüle 10 und in den Patienten wird die Seldingerkanüle 10 in proximaler Richtung aus dem Patienten herausgezogen. Dabei übt ein magnetisches Element 28 aus einem permanent magnetischen Material am rückwärtigen, nicht in den Patienten eingeführten Ende des Führungsdrahtes 12 eine Magnetkraft auf die magnetische Basis 26 des Sicherheitsclips 20 aus. Dabei zieht das magnetische Element 28 des Führungsdrahtes 12 die magnetische Basis 26 des Sicherheitsclips 20 an. Diese magnetische Wechselwirkung führt dazu, dass beim rückwärtigen Abziehen der Seldingerkanüle 10 von dem Führungsdraht 12 der Sicherheitsclip 20 in der größtmöglichen Nähe zu dem magnetischen Element 28 verbleibt, wie in Fig. 2 dargestellt. Dabei wird der Sicherheitsclip 20 in distaler Richtung, das heißt in Richtung des distalen Endes 14 der Seldingerkanüle 10 entlang des Rohres 18 verschoben. Beim vollständigen Abziehen der Seldingerkanüle 10 von dem Führungsdraht 12 wird der Sicherheitsclip 20 von der Vertiefung 30 daran gehindert, vollständig von der Seldingerkanüle 10 entfernt zu werden. Beim Anliegen der Basis 26 an der Vertiefung 30 ragen die vorderen, distalen Enden der beiden Federarme 24 über das distale Ende 14 des Rohres hinaus. Diese Lage ist in Fig. 3 dargestellt. Die Federarme 24 sind dabei in ihre natürliche Ausgangslage zurückgefedert und verdecken das distale Ende 14 der Seldingerkanüle. In der in Fig. 3 dargestellten Position schützt der Sicherheitsclip das distale Ende 14 der Seldingerkanüle, um Stichverletzungen zu vermeiden.

## Patentansprüche

1. Sicherheitsclip (20) zum Aufschieben auf eine Kanüle (10) mit einem langgestreckten, an beiden Enden offenen Rohr (18) zur Aufnahme eines langgestreckten Elements (12), wobei der Sicherheitsclip (20) zum Verschieben entlang des Rohres das Rohr außen umschließend ausgebildet ist,
**dadurch gekennzeichnet,**
**dass** der Sicherheitsclip (20) dazu ausgebildet ist, das langgestreckte Element (12) derart durch Magnetkraft anzuziehen oder abzustoßen, dass der Sicherheitsclip (20) beim Vorschieben entlang des Rohres über eines (14) der beiden Enden hinaus das Ende (14) umschließt und verdeckt.

2. Sicherheitsclip (20) nach Anspruch 1, **dadurch gekennzeichnet, dass** der Sicherheitsclip ein magnetisch mit dem langgestreckten Element (12) zusammenwirkendes Magnetelement oder ferromagnetisches Material aufweist.

3. Sicherheitsclip (20) nach Anspruch 1 oder 2, **dadurch gekennzeichnet, dass** der Sicherheitsclip (20) eine Basis und mindestens einen von der Basis abstehenden Federarm aufweist, der zum federnden Verschließen eines Rohrendes (14) beim Vorschieben über das Rohrende hinaus ausgebildet ist.

4. Sicherheitsclip (20) nach Anspruch 3, **dadurch gekennzeichnet, dass** die Basis ein magnetisch mit dem langgestreckten Element zusammenwirkendes magnetisches Element oder ferromagnetisches Material aufweist.

5. Kanüle (10), mit einem langgestreckten an beiden Enden (14, 16) offenen Rohr zur Aufnahme eines langgestreckten Elements (12) und einem das Rohr (18) umschließenden, verschiebbaren Sicherheitsclip (20) nach einem der Ansprüche 1 - 4.

6. Kanüle (10) nach Anspruch 5, **dadurch gekennzeichnet, dass** der Sicherheitsclip (20) eine Basis und mindestens einen von der Basis abstehenden Federarm aufweist, der in Richtung auf das Rohrinnere derart elastisch vorgespannt ist, dass er beim Vorschieben über das Rohrende (14) hinaus dieses automatisch verschließt.

7. Kanüle (10) nach einem der Ansprüche 5 oder 6, **dadurch gekennzeichnet, dass** die Kanüle (10) im Bereich ihres einen Endes (14) mit einem mit dem Sicherheitsclip (20) zusammengreifenden Vorsprung oder einer mit dem Sicherheitsclip (20) zusammengreifenden Vertiefung (30) versehen ist, um den Vorschub des Sicherheitsclips (20) zu stoppen.

8. Kanüle (10) nach Anspruch 7, **dadurch gekennzeichnet, dass** der Abstand des Vorsprungs oder der Vertiefung (30) von dem Kanülenende geringer ist als die Länge des Federarms.

9. Kanüle (10) nach einem der Ansprüche 5-8, **dadurch gekennzeichnet, dass** das dem einen Ende (14) gegenüberliegenden Ende (16) der Kanüle (10) mit einem vergrößerten Außendurchmesser versehen ist.

10. Kanüle (10) nach einem der Ansprüche 5 - 9, **dadurch gekennzeichnet, dass** das langgestreckte Element (12) ein Führungsdraht ist und die Kanüle (10) zur Positionierung des Führungsdrahtes in einem Gefäß eines Patienten dient.

11. Langgestrecktes Element (12) zur Verwendung mit einer Kanüle (10) nach einem der Ansprüche 5 - 10, **dadurch gekennzeichnet, dass** das langgestreckte Element (12) an seinem dem einen Ende (14) gegenüberliegenden Ende (16) dazu ausgebildet ist, mit seiner Umgebung in magnetische Wechselwirkung zu treten.

12. Langgestrecktes Element nach Anspruch 11, **dadurch gekennzeichnet, dass** das langgestreckte Element ein magnetisch mit dem Sicherheitsclip zusammenwirkendes Magnetelement oder ferromagnetisches Material aufweist.

## Claims

1. Safety clip (20) for pushing onto a cannula (10), comprising an elongated tube (18), open at both ends, for receiving an elongated element (12), the safety clip (20) being configured, so as to be displaceable along the tube, to enclose the tube on the outer side,
**characterized in that**
the safety clip (20) is configured to attract or repel the elongated element (12) by magnetic force such that the safety clip (20), as it is advanced along the tube beyond one (14) of the two ends, encloses and covers the end (14).

2. Safety clip (20) according to claim 1, **characterized in that** the safety clip comprises a magnetic element or ferromagnetic material magnetically cooperating with the elongated element (12).

3. Safety clip (20) according to claim 1 or 2, **characterized in that** the safety clip (20) has a base and at least one spring arm protruding from the base, designed to resiliently close a tube end (14) as it is advanced beyond the tube end.

4. Safety clip (20) according to claim 3, **characterized in that** the base comprises a magnetic element or ferromagnetic material magnetically cooperating with the elongated element.

5. Cannula (10) having an elongated tube, open at both ends (14, 16), for receiving an elongated element (12) and a movable safety clip (20) enclosing the tube (18) according to any of claims 1 to 4.

6. Cannula (10) according to claim 5, **characterized in that** the safety clip (20) has a base and at least one spring arm protruding from the base, the arm being elastically biased towards the tube interior such that it automatically closes the same when it is advanced beyond the tube end (14).

7. Cannula (10) according to any of claims 5 or 6, **characterized in that**, in the region of one of its ends (14), the cannula (10) is provided with a protrusion cooperating with the safety clip (20) or with a recess (30) cooperating with the safety clip (20) so as to stop the advance movement of the safety clip (20).

8. Cannula (10) according to claim 7, **characterized in that** the distance of the protrusion or the recess (30) from the cannula end is smaller than the length of the spring arm.

9. Cannula (10) according to any of claims 5 to 8, **characterized in that** the end (16) of the cannula (10) opposite the one end (14) has an enlarged outer diameter.

10. Cannula (10) according to any of claims 5 to 9, **characterized in that** the elongated element (12) is a guide wire and the cannula (10) serves to position the guide wire in a vessel of a patient.

11. Elongated element (12) for use with a cannula (10) according to any of claims 5 to 10, **characterized in that** the elongated element (12) has its end (16) opposite said one end (14) configured for magnetic interaction with its surroundings.

12. Elongated element (12) according to claim 11, **characterized in that** the elongated element comprises a magnetic element or ferromagnetic material magnetically cooperating with the safety clip.

## Revendications

1. Agrafe de sécurité (20) destinée à glisser sur une canule (10) pourvue d'un tube allongé (18) ouvert aux deux extrémités et destiné à recevoir un élément allongé (12), l'agrafe de sécurité (20) étant configurée pour être déplacée le long du tube en entourant extérieurement le tube,
**caractérisée en ce que**
l'agrafe de sécurité (20) est conçue pour attirer ou repousser l'élément allongé (12) par une force magnétique telle que l'agrafe de sécurité (20) entoure et recouvre l'extrémité (14) lorsqu'elle coulisse le long du tube au-delà de l'une (14) des deux extrémités.

2. Agrafe de sécurité (20) selon la revendication 1, **caractérisée en ce que** l'agrafe de sécurité comporte un élément magnétique ou un matériau ferromagnétique coopérant magnétiquement avec l'élément allongé (12).

3. Agrafe de sécurité (20) selon la revendication 1 ou 2, **caractérisée en ce que** l'agrafe de sécurité (20) comporte une base et au moins un bras élastique qui fait saillie de la base et qui est conçu pour fermer élastiquement une extrémité de tube (14) lors du coulissement au-delà de l'extrémité du tube.

4. Agrafe de sécurité (20) selon la revendication 3, **caractérisée en ce que** la base comporte un élément magnétique ou un matériau ferromagnétique coopérant magnétiquement avec l'élément allongé.

5. Canule (10) ayant un tube allongé ouvert aux deux extrémités (14, 16) et destiné à recevoir un élément allongé (12) et une agrafe de sécurité (20), coulissante et entourant le tube (18), selon l'une des revendications 1 à 4.

6. Canule (10) selon la revendication 5, **caractérisée en ce que** l'agrafe de sécurité (20) comporte une base et au moins un bras élastique qui fait saillie de la base et qui est précontraint élastiquement en direction de l'intérieur du tube de manière à fermer automatiquement l'extrémité de tube (14) lors du coulissement au-delà celle-ci.

7. Canule (10) selon l'une des revendications 5 ou 6, **caractérisée en ce que** la canule (10) est pourvue, au niveau d'une extrémité (14), d'une saillie s'engageant avec l'agrafe de sécurité (20) ou d'un évidement (30) s'engageant avec l'agrafe de sécurité (20) pour arrêter le coulissement de l'agrafe de sécurité (20).

8. Canule (10) selon la revendication 7, **caractérisée en ce que** la distance entre la saillie ou l'évidement (30) et l'extrémité de la canule est inférieure à la longueur du bras élastique.

9. Canule (10) selon l'une des revendications 5 à 8, **caractérisée en ce que** l'extrémité (16), opposée à l'extrémité (14), de la canule (10) est pourvue d'un diamètre extérieur agrandi.

10. Canule (10) selon l'une des revendications 5 à 9, **caractérisée en ce que** l'élément allongé (12) est un fil de guidage et la canule (10) est destinée à positionner le fil de guidage dans un vaisseau d'un patient.

11. Élément allongé (12) destiné à être utilisé avec une canule (10) selon l'une des revendications 5 à 10, **caractérisé en ce que** l'élément allongé (12) est conçu à son extrémité (16), opposée à l'extrémité (14), de manière à entrer en interaction magnétique avec son environnement.

12. Élément allongé selon la revendication 11, **caractérisé en ce que** l'élément allongé comporte un élément magnétique ou un matériau ferromagnétique coopérant magnétiquement avec l'agrafe de sécurité.
